Europäisches Patentamt

European Patent Office

Office européen des brevets

(11) Publication number: **0 276 542 B1**

(19)

(12) # EUROPEAN PATENT SPECIFICATION

(45) Date of publication of patent specification: **29.04.92**  (51) Int. Cl.5: **B01D 3/00**, C01B 7/19

(21) Application number: **87308855.3**

(22) Date of filing: **06.10.87**

(54) **Distillation method and apparatus.**

(30) Priority: **27.01.87 JP 15219/87**

(43) Date of publication of application:
**03.08.88 Bulletin  88/31**

(45) Publication of the grant of the patent:
**29.04.92 Bulletin  92/18**

(84) Designated Contracting States:
**DE FR GB**

(56) References cited:
**CH-A- 269 497**
**US-A- 2 167 395**
**US-A- 3 325 376**

(73) Proprietor: **TAMA CHEMICALS CO., LTD.**
**36-2, Kamata 5-chome Ohta-ku**
**Tokyo(JP)**

Proprietor: **MOSES LAKE INDUSTRIES, INC.**
**780 Randolph Road**
**Moses Lake Washington 98837(US)**

(72) Inventor: **Shimizu, Shumpei**
**20 Fairway Drive**
**Moses Lake Washington 98837(US)**
Inventor: **Yoshizako, Mamoru**
**131-21, Miwa-chou**
**Machida-shi Tokyo(JP)**
Inventor: **Cho, Toshitsura**
**1-18, Takaishi 5-chome Asou-ku**
**Kawasaki-shi Kanagawa-ken(JP)**

(74) Representative: **Cockbain, Julian Roderick**
**Michaelson et al**
**Frank B. Dehn & Co. European Patent Attorneys Imperial House 15-19, Kingsway**
**London WC2B 6UZ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 276 542 B1

**Description**

The present invention relates to a distillation method for producing chemicals at high levels of purity and, more particularly, it relates to a method of and an apparatus for producing such chemicals by separating out therefrom non-volatile impurities which may be contained in trace amounts.

With recent developments in semi-conductor, optical fibre, fine ceramic and other industries, higher levels of purity have been more and more in demand by these industries for the materials they use, in particular higher degrees of purity with respect to non-volatile impurities such as metal impurities and high molecular weight impurities. Furthermore, with the development of analytical equipment and the constitution of an analytic chamber as a clean room, analysis and microanalysis for several metal elements to parts per billion (ppb) or even parts per trillion (ppt) levels has become possible. Naturally, high purity has been demanded for various starting chemicals for producing the materials used by such industries and for various analytical reagents used for analysis and microanalysis.

Where the chemicals which are required to be in highly pure form are liquid and thermally stable, a distillation purification method has usually been employed in their preparation. Thus, for example, Japanese Patent Laid-Open Publication No. 191502/1986 describes a method of purifying hydrofluoric acid containing arsenic impurities which involves adding a halogen fluoride to the hydrofluoric acid to react with the arsenic compounds and subsequently purifying by distillation.

Purification by the distillation method involves heating a liquid to its boiling point and then cooling to condense the evolved vapour. Where purification to a high level is desired, the procedure may involve repeating the distillation or conducting rectification, a procedure which can achieve the same effect as redistillation in one distillation step by enlarging the section of the distillation apparatus between the heating portion and the condensing portion in which contact between the vaporous distillate and the liquid occurs.

If the leaching of impurities from the distillation apparatus were to be fully eliminated by the selection of the material it is made from and if there were to be no droplet entrainment by means of which minute droplets are dispersed in the vaporous distillate and carried onto the condensing section by the gas flowing through the distillation apparatus, the purity of the chemical so produced, with respect to non-volatile impurities, would be improved as far as is possible by the distillation purification method and it would be possible to produce chemicals at super high purities, for example of ppb or ppt levels relative to metal impurities.

However, in the distillation purification method the droplet entrainment phenomenon cannot be completely eliminated by conventional means even if for instance rectification is employed. If it is intended to reduce the droplet entrainment phenomenon to yield chemicals at a super high purity, then conventional distillation or rectification would have to be carried out at a rate so reduced that the yield per unit time would be decreased to a level which is extremely uneconomical from an industrial and commercial point of view.

Thus, at present, a compromise has to be made between the production rate and the degree of purity obtained and whenever super high purity is demanded, for example for analytical reagents, small amounts of the chemicals have had to be purified individually in a laboratory.

Accordingly, it is an object of the present invention to provide a method and apparatus for distillation for reducing non-volatile impurity levels.

We have now found that the level of non-volatile impurities in a distillate may be substantially reduced by passing the distillate, while in the vapour state, through a contact section heated to a temperature above the boiling point of the distillate.

Viewed from one aspect, the invention thus provides a method of distillation to reduce non-volatile impurity levels, characterised in that before being condensed a vaporous distillate is passed through a contact section heated to a temperature higher than the boiling point of said distillate whereby to evaporate droplets entrained in said vaporous distillate.

Viewed from another aspect, the invention provides a method of producing chemicals at high purity by distilling a material to be distilled in a distillation device comprising a heating section for heating the material to be distilled, a contact section including contact members with which vapours of evaporated materials to be distilled are brought into contact, and a condensing section for condensing vapours passing through the contact section, thereby eliminating non-volatile impurities in the material to be distilled, wherein the contact section is heated at least to a temperature higher than the boiling point of the material to be distilled.

Viewed from a still further aspect, the invention provides a distillation apparatus for distillation to reduce non-volatile impurity levels, said apparatus comprising a heating section for evaporating material to be distilled, a contact section having contact surfaces for contacting vaporous distillate in said contact section,

2

and a condensation section for condensing vaporous distillate which has passed through the said contact section, characterised in that said apparatus further comprises heating means arranged to heat said contact section to a temperature higher than the boiling point of the vaporous distillate passing therethrough whereby to evaporate droplets entrained in said vaporous distillate.

Viewed from a yet still further aspect, the invention also provides an apparatus for producing chemicals at high purity, that is a distillation apparatus comprising a heating section for heating material to be distilled, a contact section including contact members to be in contact with the vapours of evaporated material to be distilled, and a condensing section for condensing the vapours passing through the contact section, wherein a heating means is disposed at the contact section for heating the contact section at least to a temperature higher than the boiling point of the material to be distilled.

For the method and in the apparatus of the present invention, the heating section for heating the material to be distilled may be the same as that used for heating the material to be distilled in conventional distillation apparatus, the same contact section as is employed in ordinary distillation apparatus may also be used, and furthermore the same condensing section as is usually in used in conventional distillation apparatus may also be used.

Referring to the contact section, the vapour contacting surfaces may for example be provided by the surfaces of contact members disposed in the contact section, for example trays (e.g. of perforated or bubble plates) or particulate packing of appropriate size and shape (such as broken pieces, spheres, raschig rings, saddles, etc). The vapour contacting surfaces of the contact members should however be inert with respect to the material to be distilled, at least under the distillation conditions. As the material for the contact member, there can be mentioned silica gel, alumina, Teflon®, glass, synthetic quartz, high purity graphite and various kinds of thermally and chemically stable plastics; depending upon the nature of the material to be distilled an appropriate material for the contact member may be selected accordingly.

The contact section can be heated to the required temperature by a heating means, for example externally disposed heating means such as electrically resistive heat generation means, IR lamps, hot air blowers and the like, or heating may be by means of direct current supply to a contact member disposed in the contact section or by high frequency heating means such a high frequency induction heating and high frequency dielectric heating.

In this invention, the degree and manner of heating in the heating section and in the contact section may be set as desired as long as the vaporous distillate is caused to pass through the contact section, for example at a predetermined velocity, there to be heated to a temperature higher than the boiling point of the material being distilled. The heating temperature in the contact section will basically be such that the material to be distilled can be completely evaporated there; usually it is desirable to heat to a temperature higher by about 1 to 200 C°, preferably by about 1 to 100 C°, than the boiling point of the material to be distilled. Furthermore, the velocity at which the vaporous distillate passes through the contact section (upon which the distillation rate is dependent) is preferably from 0.01 to 10 s$^{-1}$, more preferably 0.05 to 5 s$^{-1}$, as expressed in terms of the flow rate per unit volume in the contact section, that is the space velocity (SV). However, it will be noted that if the temperature difference between the heating temperature at the contact section and the boiling point of the material to be distilled is insufficient, then droplets entrained in the vaporous distillate entering the contact section may not be completely evaporated at the surfaces of the contact members and thus the entrainment phenomenon will not be completely eradicated, and if, the temperature difference is greater than 200 C° then not only may the increase in impurity vapour pressure become troublesome but the undue energy expenditure will become uneconomical. On the other hand referring to the velocity of the vaporous distillate passing through the contact section, reduced velocities will result in the purification requiring longer periods of time and increased velocities will result in reduced heat exchanging efficiency in the contact section thereby resulting in uneconomical energy use, and furthermore at excessively fast velocities the entrainment phenomenon cannot be entirely eliminated.

As the material to be distilled, the material to be purified by the method and apparatus of the present invention, any material may be used as long as it can be purified by distillation; preferred materials include those which are limited in the improvement of purity by the entrainment phenomenon. Particularly suitable specific examples can include, for example, inorganic acids such as hydrochloric acid, nitric acid, hydrofluoric acid, perchloric acid, hydroiodic acid, organic acids such as acetic acid and formic acid, organic solvents such as acetone, chloroform, trichloroethylene and carbon tetrachloride, as well as water.

The non-volatile impurities whose levels can be reduced by use of the method and apparatus of the present invention will generally be impurities which are introduced in trace amounts in the production of the material to be distilled and they can include, for example, metal impurities such as aluminium (Al), iron (Fe), calcium (Ca), copper (Cu), magnesium (Mg), managnese (Mn), sodium (Na) and potassium (K) or various high molecular impurities of unknown structures.

Where the purification method and apparatus of the invention are to be used to produce chemicals of super high purification, i.e. down to ppb or ppt levels, the apparatus is desirably installed entirely within a clean room with the receiver for the liquid condensate being operated in a claean bench.

The method and apparatus according to the present invention may be operated batchwise or continuously, for example depending on the nature and the quantity of the material to be purified by distillation or on the degree of purity that is required.

In the operation of the present invention, since droplets entrained in the vaporous distillate are completely evaporated to leave the non-volatile impurities from the droplets in the heated contact section, the non-volatile impurities can thus be collected in the contact section and, as a result, the purity of the distilled liquid condensed in the condensing section is improved.

The method and apparatus according to the invention thus allow easy production at a reduced cost of high purity chemicals, especially chemicals which are purified to a high degree of purity with respect to non-volatile impurities, particularly metal impurities or high molecular weight impurities.

The method and apparatus of the invention are thus particularly suitable for producing chemicals at high purity, for example for producing highly pure starting chemicals as required for the continuously developing new technological fields, and for producing analytical reagents at the high degrees of purity required for analysis and microanalysis.

Embodiments of the present invention will now be described further in the following non-limiting Examples and with reference to the accompanying drawings, in which:-

Figure 1 is a schematic cross-sectional view of the contact section of a distillation apparatus according to the invention; and

Figure 2 is a schematic cross-sectional view of the contact section of a further distillation apparatus according to the invention.

## Example 1 - Hydrochloric Acid Purification

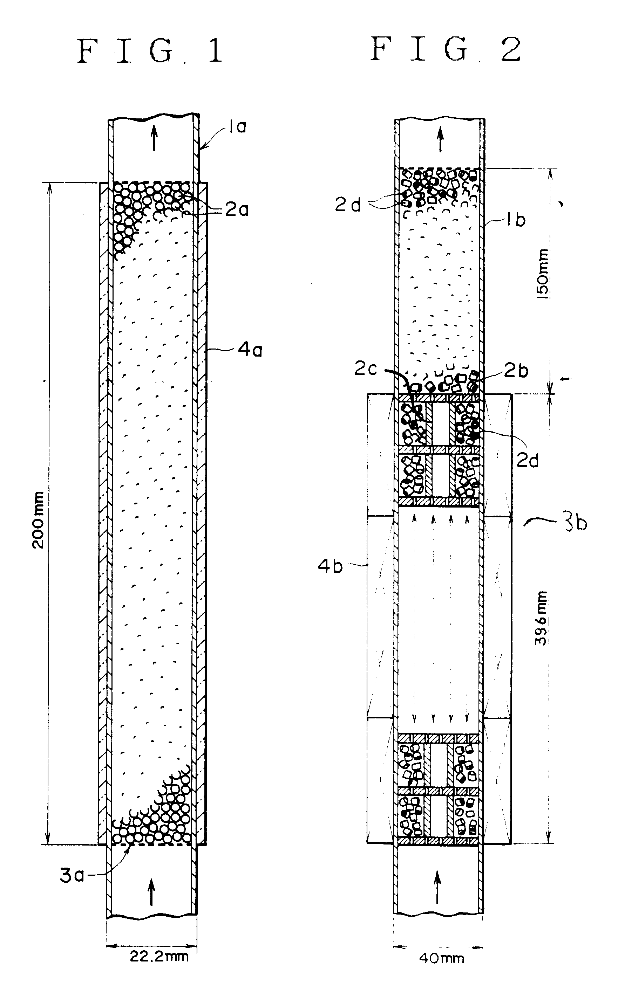

Referring to Figure 1, a quartz column 1a of 350 mm length $\times$ 22.2 mm diameter was connected to a 2 litre volume round bottom flask (not shown) made of Pyrex®. 12g of silica gel packing 2a (2mm grain size pulverization products) were packed as the contact members into a 200 mm length of the quartz column 1a, which length constituted a contact section 3a. Band heater 4a was wound around the outside of the contact section 3a of the column 1a in order to act as the heating means and to enable heating of the contact section. A Teflon® tube (not shown) having a cooler was connected to the top of the quartz column 1a and a 2 litre volume receiver made of Teflon® was connected to the top end of the Teflon® tube, the receiver being installed at a Class 100 clean bench and the entire apparatus thus assembled being installed in a clean room.

High purity hydrochloric acid was prepared by charging 1.5 liters of 20.22 weight % hydrochloric acid (containing Al, Cu, Mg, Mn, Na and K each at 50 ppm (50,000 ppb) as metal impurities) into the round bottom flask. The contact section 3a was heated by band heater 4a to 140± 5 °C which is higher by about 31 C° than the boiling point of hydrochloric acid (108.58°C)) and the round bottom flask was immersed in a hot medium bath and the space velocity in the contact section 3a and the distillation rate were set to 0.75 $s^{-1}$ and 100ml/h.

100ml of the pre-fraction of the distillation product were removed and thereafter 700ml of the distillation product were collected in a receiver and the contents of the metal impurities Al, Fe, Ca, Cu, Mg, Mn, Na and K in the high purity hydrochloric acid thus obtained were measured using a flameless electron absorption device. The results are shown in Table 1 below.

## Comparative Example 1

20.22 weight % hydrochloric acid was purified in the manner described in Example 1 above, except that contact section 3a was not heated. The contents of the metal impurities Al, Fe, Ca, Cu, Mg, Mn, Na and K in the high purity hydrochloric acid thus obtained were measured using the flameless electron absorption device. The results are shown in Table 1 below.

TABLE 1

| Impurity metal | Impurity content (ppb) | |
|---|---|---|
| | Example 1 | Comparative Example 1 |
| Al | <0.050 | 2.50 |
| Fe | 0.057 | 3.10 |
| Ca | <0.020 | 2.80 |
| Cu | 0.020 | 3.00 |
| Mg | <0.005 | 3.50 |
| Mn | <0.005 | 2.40 |
| Na | <0.005 | 2.80 |
| K | <0.005 | 2.50 |

Example 2 - Hydrofluoric Acid Purification

Referring to Figure 2, a Teflon® column 1b of 40 mm diameter was connected to a 2 liter volume still (not shown) also made of Teflon®. Perforated carbon plates 2b (of 12 mm thickness and 38 mm diameter) and Teflon® tubes 2c (of 20 mm length and 20 mm diameter) were alternately stacked within the Teflon® column 1b in order to serve as contact members, the stacking being for a total of 12 stages over a 396 mm length of the Teflon® column 1b. Teflon® Raschig rings 2d (of 5 mm outer diameter, 3 mm inner diameter and 5 mm length) were packed into the column within the plate and tube stacked portion and for a height of 150 mm thereover, there also to serve as contact members and to define the contact section 3b. A heating section 4b of a high frequency induction heating device (electronic range) was attached as the heating means to the outside of the contact section 3b to enable heating thereof to be effected. A Teflon® tube (not shown) equipped with a cooler was connected to the top of the Teflon® column 1b, a 2 liter volume receiver made of Teflon® was connected to the top end of the Teflon® tube, the receiver was set on a class 100 type clean bench and the entire apparatus thus assembled was installed within a clean room.

High purity hydrofluoric acid was prepared analogously to Example 1 by charging 1.5 liters of 38.2 weight % hydrofluoric acid (containing Al, Cu, Mg, Mn, Na and K, each at 50 ppm (50,000 ppb) as metal impurities) in the still, heating the contact section 3b using the heating section 4b of the high frequency induction heating device to 160 ± 5°C (which is higher by about 47.8°C than the boiling point of hydrofluoric acid (112.2°C)), heating the still by immersing it in a hot medium bath and setting the space velocity in the contact section 3b and the distillation rate to 0.1 s$^{-1}$ and 100 ml/h.

As in Example 1, 100 ml of the pre-distillation fraction of the distillation product were removed and thereafter 700 ml of the distillation product were collected in the receiver and the contents of metal impurities Al, Fe, Ca, Cu, Mg, Mn, Na and K in the high purity hydrofluoric acid thus obtained were measured using the flameless electron absorption device. The results are shown in Table 2 below.

Comparative Example 2

38.2 weight % hydrofluoric acid was purified in the manner described in Example 2 above, except that the contact section 3b was not heated. The contents of the metal impurities Al, Fe, Ca, Cu, Mg, Mn, Na and K in the high purity hydrofluoric acid thus obtained were measured using the flameless electron absorption device. The results are shown in Table 2 below.

TABLE 2

| Impurity metal | Impurity content (ppb) | |
|---|---|---|
| | Example 2 | Comparative Example 2 |
| Al | <0.050 | 3.75 |
| Fe | 0.035 | 4.65 |
| Ca | <0.020 | 4.20 |
| Cu | 0.015 | 4.50 |
| Mg | <0.005 | 5.25 |
| Mn | <0.005 | 3.60 |
| Na | <0.005 | 4.20 |
| K | <0.005 | 3.75 |

## Claims

1.  A method of distillation to reduce non-volatile impurity levels, characterized in that before being condensed a vaporous distillate is passed through a contact section (3a,3b) heated to a temperature higher than the boiling point of said distillate whereby to evaporate droplets entrained in said vaporous distillate.

2.  A method as claimed in claim 1 wherein said contact section (3a,3b) is heated to a temperature of 1 to 200 C° higher than said boiling point.

3.  A method as claimed in claim 1 wherein said contact section (3a,3b) is heated to a temperature about 30 to 100 C° higher than said boiling point.

4.  A method as claimed in any one of claims 1 to 3 wherein said vaporous distillate is passed through said contact section (3a,3b) at a space velocity of 0.01 to 10 $s^{-1}$.

5.  A method as claimed in any one of claims 1 to 3 wherein said vaporous distillate is passed through said contact section (3a,3b) at a space velocity of 0.05 to 5 $s^{-1}$.

6.  A distillation apparatus for carrying out the method according to Claims 1 to 5 comprising a heating section for evaporating material to be distilled, a contact section (3a,3b) having contact surfaces for contacting vaporous distillate in said contact section, and a condensation section for condensing vaporous distillate which has passed through said contact section, characterized in that said apparatus further comprises heating means (4a,4b) arranged to heat said contact section (3a,3b) to a temperature higher than the boiling point of the vaporous distillate passing therethrough whereby to evaporate droplets entrained in said vaporous distillate.

7.  Apparatus as claimed in claim 6 wherein said heating means (4a,4b) is disposed externally about said contact section (3a,3b).

## Revendications

1.  Procédé de distillation pour réduire les taux d'impuretés non-volatiles, caractérisé par le fait qu'avant qu'il ne soit condensé, on fait passer un distillat à l'état de vapeur à travers une section de contact (3a, 3b), chauffée à une température supérieure au point d'ébullition dudit distillat, ce qui permet d'évaporer des gouttelettes entraînées dans ledit distillat à l'état de vapeur.

2.  Procédé selon la revendication 1, dans lequel on chauffe ladite section de contact (3a, 3b) à une température de 1 à 200°C au-dessus dudit point d'ébullition.

3.  Procédé selon la revendication 1, dans lequel on chauffe ladite section de contact (3a, 3b) à une température d'environ 30 à 100°C au-dessus dudit point d'ébullition.

**4.** Procédé selon l'une quelconque des revendications 1 à 3, dans lequel on fait passer ledit distillat à l'état de vapeur à travers ladite section de contact (3a, 3b) à une vitesse spatiale de 0,01 à 10 s$^{-1}$.

**5.** Procédé selon l'une quelconque des revendications 1 à 3, dans lequel on fait passer ledit distillat à l'état de vapeur à travers ladite section de contact (3a, 3b) à une vitesse spatiale de 0,05 à 5 s$^{-1}$.

**6.** Appareil de distillation pour la mise en oeuvre du procédé tel que défini aux revendications 1 à 5, comprenant une section de chauffage pour évaporer la matière à distiller, une section de contact (3a, 3b) ayant des surfaces de contact pour la mise en contact du distillat à l'état de vapeur dans ladite section de contact, et une section de condensation pour condenser le distillat à l'état de vapeur qui est passé à travers ladite section de contact, caractérisé par le fait que ledit appareil comprend en outre un moyen de chauffage (4a, 4b) disposé pour chauffer ladite section de contact (3a, 3b) à une température supérieure au point d'ébullition du distillat à l'état de vapeur passant à travers elle, ce qui permet d'évaporer des gouttelettes entraînées dans ledit distillat à l'état de vapeur.

**7.** Appareil selon la revendication 6, dans lequel ledit moyen de chauffage (4a, 4b) est disposé extérieurement autour de ladite section de contact (3a, 3b).

**Patentansprüche**

**1.** Destillationsverfahren zum Vermindern des Gehalts an nicht flüchtiger Verunreinigung, dadurch gekennnzeichnet, daß ein dampfförmiges Destillat, bevor es kondensiert wird, durch eine Kontaktzone (3a, 3b) geleitet wird, die auf eine Temperatur oberhalb des Siedepunkts dieses Destillats erhitzt ist, so daß in diesem dampfförmigen Destillat mitgeschleppte Tröpfchen verdampft werden.

**2.** Verfahren gemäß Anspruch 1, bei dem diese Kontaktzone (3a, 3b) auf eine Temperatur, die um 1 bis 200°C oberhalb dieses Siedepunkts liegt, erhitzt ist.

**3.** Verfahren gemäß Anspruch 1, bei dem diese Kontaktzone (3a, 3b) auf eine Temperatur erhitzt ist, die etwa 30 bis 100°C höher als dieser Siedepunkt liegt.

**4.** Verfahren gemäß einem der Ansprüche 1 bis 3, bei dem das dampfförmige Destillat mit einer Raumgeschwindigkeit von 0,01 bis 10 s$^{-1}$ durch diese Kontaktzone (3a, 3b) geleitet wird.

**5.** Verfahren gemäß einem der Ansprüche 1 bis 3, bei dem das dampfförmige Destillat mit einer Raumgeschwindigkeit von 0,05 bis 5 s$^{-1}$ durch diese Kontaktzone (3a, 3b) geleitet wird.

**6.** Destillationsvorrichtung zur Durchführung des Verfahrens nach Ansprüchen 1 bis 5, die eine Heizzone zum Verdampfen des zu destillierenden Materials, eine Kontaktzone (3a, 3b) mit Kontaktflächen zum Kontaktieren von dampfförmigem Destillat in dieser Kontaktzone, und eine Kondensationszone zum Kondensieren von dampfförmigem Destillat, welches diese Kontaktzone passiert hat, aufweist, dadurch gekennzeichnet, daß die Vorrichtung außerdem eine Heizeinrichtung (4a, 4b) aufweist, die so angeordnet ist, daß sie die Kontaktzone (3a, 3b) auf eine Temperatur oberhalb des Siedepunkts des durch diese geleiteten dampfförmigen Destillats erhitzt, wodurch in diesem dampfförmigen Destillat mitgerissene Tröpfchen verdampft werden.

**7.** Vorrichtung gemäß Anspruch 6, in der die Heizeinrichtung (4a, 4b) außen an dieser Kontaktzone (3a, 3b) angeordnet ist.

# F I G . 1

# F I G . 2